# EUROPEAN PATENT APPLICATION

(11) **EP 0 676 412 A1**
(43) Date of publication of application: **11.10.1995**
(21) Application number: 94930355.6
(22) Date of filing: 24.10.1994
(51) Int. Cl.: C07K 14/47, C12P 21/02, G01N 33/53

(54) **PURIFIED HUMAN CHONDROCALCIN, PROCESS FOR PRODUCING THE SAME, AND UTILIZATION THEREOF**

(30) Priority: 25.10.1993 JP 287275/93
(71) Applicant: TEIJIN LIMITED, Osaka-shi Osaka 541 (JP)
(72) Inventor: KOBAYASHI, Shinji, Tokyo Res. Cter. of Teijin Ltd., Hino-shi, Tokyo 191 (JP); EGUCHI, Hiroshi, Tokyo Res. Center of Teijin Ltd., Hino-shi, Tokyo 191 (JP); HOSODA, Kenji, Tokyo Res. Center of Teijin Ltd., Hino-shi, Tokyo 191 (JP); NAKAMOTO, Tadakatsu, Iwakuni Res. Center of, Iwakuni-shi, Yamaguchi 740 (JP)
(74) Representative: Votier, Sidney David
(86) International application number: JP9401780
(87) International publication number: WO9511920

(57) **Abstract**

A purified human chondrocalcin produced by gene recombination techniques, a process therefor, and a reagent and kit for immunoassaying a human chondrocalcin in a specimen by using the purified chondrocalcin.

## Description

### Technical Field

This invention relates to purified human chondrocalcin, a process for preparation thereof and utilization thereof. More specifically, this invention relates to purified human chondrocalcin obtained by genetic recombination technique, a process for preparation thereof and utilization thereof.

### Background Art

Chondrocalcin is a protein first separated and isolated from the epiphysial cartilage of a bovine fetus in 1983 by H. U. Choi et al., and is known to be a molecule having a molecular weight of about 110,000, consisting of three subunits each having a molecular weight of about 35,000 [Christopher Niyibizi et al.; Biochimica et Biophysica Acta, 916, 493-499 (1987)].

As to the distribution of this protein in living bodies, a histochemical research using mainly cattle is reported by A. R. Poole et al. [A. R. Poole et al., J. Cell Biol., 98, 54-65 (1984); Clinical Orthopaedics and Related Research, 208, 114-118 (1986)]. It is stated therein that chondrocalcin exists in the largest amount in cartilages, particularly deep sites of cartilages, where calcification is progressing, and does not exist in mature bones, teeth and other tissues.

It is surmised from the above characteristic of the above distribution in living bodies and from the fact that this protein binds to calcium ions and hydroxyapatite, that the physiological actions of chondrocalcin is deeply involved with calcification of cartilages. Immunological methods such as tissue staining are frequently used as experimental means in these researches, and therein, tissues of bovine are researched using polyclonal antibodies obtained by immunizing rabbits with bovine chondrocalcin.

On the other hand, it is clarified by M. VAN DER REST et al. that chondrocalcin is identical to C-propeptide of type II procollagen [Michel VAN DER REST et al.; Biochem. J. 237, 923-925 (1986)]. Namely, it is ascertained by REST et al. that bovine chondrocalcin is a protein having the same amino acid sequence as C-propeptide of bovine type II procollagen. However, its specific whole amino acid sequence is not disclosed in this literature.

In Proc. Natl. Acad. Sci. U.S.A., Vol. 82, pp.2555-2559, May 1985 Biochemistry, published prior thereto, Kathryn S.E. Cheah et al. made analysis of human type II collagen gene (COL2AI), and presented the amino acid sequence of human type II collagen.

Accordingly, it is concluded from the above two literatures that human chondrocalcin has been identical to C-propeptide of human type II procollagen and its whole amino acid sequence has been clarified.

On the other hand, various attempts have been reported on expression of procollagen by recombinant technique.
(1) First, K. J. Doege et al. reported on in vitro reassembly of chicken type I procollagen as follows. When carboxyl peptides (this contains carboxyl telopeptide and carboxyl propeptide) obtained by digesting chicken type I procollagen with collagenase were reassembled, a trimer was formed, whereas when the carboxyl propeptide (propeptidase-cleaved C-peptide) part alone was reassembled, a trimer was not formed.
   Based on the above study, they reported that the existence of carboxyl telopeptide is important for trimerization of carboxyl peptides [Kurt J. Doege et al.; The Journal of Biological Chemistry, Vol.261, No.19, pp.8924-8935 (1986)].
(2) L. Ala-Kokko et al. reported on expression of human type II procollagen in mouse NIH3T3 cell [Leena Ala-Kokko et al.; The Journal of Biological Chemistry, Vol.266, No.22, pp.14175-14178 (1991)]. In this literature, it is ascertained, using a rabbit polyclonal antibody obtained by immunizing a rabbit with the synthetic peptide consisting of 23-residues existing in the telopeptide at the C-terminus of human type II procollagen, that the above expressed human type II procollagen is a trimer.
(3) A. L. Sieron et al. reported results obtained by expressing a mutant of human type II procollagen wherein the telopeptide of human type II procollagen was made to remain but the intermediate helix part was deleted, in mouse NIH3T3 cell, and examining the mutant for heat stability [Aleksander L. Sieron et al.; The Journal of Biological Chemistry, Vol.268, No.28, pp.21232-21237 (1993)].

There is no disclosure in these literatures of (2) and (3) about procuring fragments of carboxyl propeptide from the obtained human type II procollagen or its mutant. However, it is surmised from the above literature of K. J. Doege et al. that even if this human type II procollagen or its mutant were digested with collagenase, fragments containing carboxyl propeptide would contain carboxyl telopeptide and hence, a fragment consisting of carboxyl propeptide alone (i.e., chondrocalcin) could not be obtained.

On the other hand, there have been only a few reports of pathological researches on chondrocalcin, and future development is expected. A. R. Poole et al. state that they are paying attention to chondrocalcin in association with pathology in diseases related to growth, endochondral ossification and abnormal calcification, etc. [A. R. Poole et al., Clinical Orthopaedics and Related Research, 208, 114-118 (1986)].

Shinmei et al. reported that they had assayed chondrocalcin in articular humor, and chondrocalcin could be utilized as a marker for early diagnosis and/or judgment of therapeutic effects of articular pathosis such as osteoarthritis and traumatic hydrarthrosis [Rinsho Kensa, Vol. 35, No. 13, pp. 1293-1298 (1991)].

Therefore, it plays a very important role on researches in fundamental medicine and clinical medicine of chondrocalcin to assay chondrocalcin. A. Hinek et al. assayed chondrocalcin in cartilage extracts and culture supernatants of chondrocytes by radioimmunoassay in 1987 [A. Hinek et al., J. Cell Biol., 104, 1435-1441 (1987)].

European Patent No. 0425665A1 discloses a method which comprises immunologically assaying the amount of chondrocalcin in humor of a mammal using an anti-chondrocalcin antibody. Anti-chondrocalcin antibodies specifically used in this immunological assay are a monoclonal antibody and a polyclonal antibody which are obtained using bovine chondrocalcin as an immunogen and recognize human chondrocalcin. Chondrocalcin actually used for making a calibration curve in this immunological assay is purified chondrocalcin derived from bovine.

As to chondrocalcin for constructing these assay systems, although there is an example reporting purification of chondrocalcin from bovine, it has not been accomplished to artificially synthesize natural-type chondrocalcin where a perfect trimer is formed. Particularly, as to human chondrocalcin, taking its sample itself is difficult, and a purified product from a human being is not even obtained.

On the other hand, even if, in order to synthesize human chondrocalcin using a genetic engineering method, human type II procollagen or a mutant thereof were first expressed according to the method of Ala-Kokko et al. or the method of A. L. Sieron et al. and then these were digested with collagenase, the resultant product would be only carboxy propeptide containing carboxy telopeptide, and thus it is impossible to think that a protein (chondrocalcin) consisting solely of carboxy propeptide can be obtained.

Further, even if a cell were produced in which a DNA sequence encoding an amino acid sequence constituting human chondrocalcin was recombined and proteins were expressed, a means would be unclear whereby such a protein having a high-order structure is efficiently expressed and purified chondrocalcin not containing other proteins is obtained, because chondrocalcin has a trimer structure containing many S-S bonds. Further, such an attempt has not been made.

### Disclosure of Invention

Therefore, the first object of this invention lies in providing purified human chondrocalcin obtained according to a genetic engineering method.

The second object of this invention lies in providing human chondrocalcin capable of immunologically reacting with an anti-bovine chondrocalcin antibody, thus human chondrocalcin usable as a reagent for immunological assay of human chondrocalcin in human specimens.

The third object of this invention lies in providing a cell line and a process, whereby purified human chondrocalcin can be prepared by a genetic engineering method.

Another object of this invention lies in providing an industrially advantageous process for preparing purified human chondrocalcin.

Still another object of this invention lies in providing a reagent and a kit for immunologically assaying human chondrocalcin in human specimens utilizing purified human chondrocalcin.

According to researches by the present inventors, the above objects of this invention can be attained by providing purified human chondrocalcin comprising a trimer of a polypeptide having the following amino acid sequence [I].

Xₘ - P [I]

wherein
- X: denotes an amino acid residue derived from a cleavage sequence,
- m: denotes 0 or 1, and
- P: denotes the following amino acid sequence:

Asp Gln Ala Ala Gly Gly Leu Arg Gln His Asp Ala Glu Val Asp
1               5                   10                  15
Ala Thr Leu Lys Ser Leu Asn Asn Gln Ile Glu Ser Ile Arg Ser
                20                  25                  30
Pro Glu Gly Ser Arg Lys Asn Pro Ala Arg Thr Cys Arg Asp Leu
                35                  40                  45
Lys Leu Cys His Pro Glu Trp Lys Ser Gly Asp Tyr Trp Ile Asp
                50                  55                  60
Pro Asn Gln Gly Cys Thr Leu Asp Ala Met Lys Val Phe Cys Asn
                65                  70                  75
Met Glu Thr Gly Glu Thr Cys Val Tyr Pro Asn Pro Ala Asn Val
                80                  85                  90
Pro Lys Lys Asn Trp Trp Ser Ser Lys Ser Lys Glu Lys Lys His
                95                  100                 105
Ile Trp Phe Gly Glu Thr Ile Asn Gly Gly Phe His Phe Ser Tyr
                110                 115                 120
Gly Asp Asp Asn Leu Ala Pro Asn Thr Ala Asn Val Gln Met Thr
                125                 130                 135
Phe Leu Arg Leu Leu Ser Thr Glu Gly Ser Gln Asn Ile Thr Tyr
                140                 145                 150

His Cys Lys Asn Ser Ile Ala Tyr Leu Asp Glu Ala Ala Gly Asn
                155                 160                 165
Leu Lys Lys Ala Leu Leu Ile Gln Gly Ser Asn Asp Val Glu Ile
                170                 175                 180
Arg Ala Glu Gly Asn Ser Arg Phe Thr Tyr Thr Ala Leu Lys Asp
                185                 190                 195
Gly Cys Thr Lys His Thr Gly Lys Trp Gly Lys Thr Val Ile Glu
                200                 205                 210
Tyr Arg Ser Gln Lys Thr Ser Arg Leu Pro Ile Ile Asp Ile Ala
                215                 220                 225
Pro Met Asp Ile Gly Gly Pro Glu Gln Glu Phe Gly Val Asp Ile
                230                 235                 240
Gly Pro Val Cys Phe Leu
                245

Further, according to researches by the present inventors, it was found that the above purified human chondrocalcin can be obtained by a process for preparing purified human chondrocalcin which comprises growing an eucaryote cell transformed or infected with a vector or virus containing a sequence which contains
(a) an eucaryotic promoter;
(b) optionally, an enhancer stimulating the above eucaryotic promoter; and
(c) the following DNA sequence [II] transcribed by the above eucaryotic promoter

   B-(C)ₘ-D [II]

   wherein
   - B: denotes a DNA sequence encoding an eucaryotic signal peptide,
   - C: denotes a DNA sequence encoding the amino acid residue X in the above formula [I].
   - m: denotes 0 or 1,
   - D: denotes a DNA sequence encoding the amino acid sequence P in the above formula [I],
   these sequences being arranged such that they can express human chondrocalcin,
   and isolating secreted or accumulated human chondrocalcin.

This invention is described in more detail below.

The human chondrocalcin of this invention is a trimer of a polypeptide having the above amino acid sequence [I], and its molecular weight is in the range of about 100,000 to about 120,000. Such range of molecular weight arises from the fact that the kinds of sugar chains added for modification differ depending on hosts used for the expression. The molecular weight can be ascertained by subjecting it to SDS-PAGE under a non-reducing condition. An example of the SDS-PAGE condition is as follows.

### Condition of SDS-PAGE

To a sample solution is added an equal amount of the following sample preparation solution,
5mM Tris-HCl buffer pH 6.8
10 % glycerol
0.01 % Bromophenol Blue
1 % SDS,
and the mixture is subjected to incubation at 95° C for 5 minutes, and then electrophoresed at 30 mA for 90 minutes. The gel is stained with Coomassie Brilliant Blue.

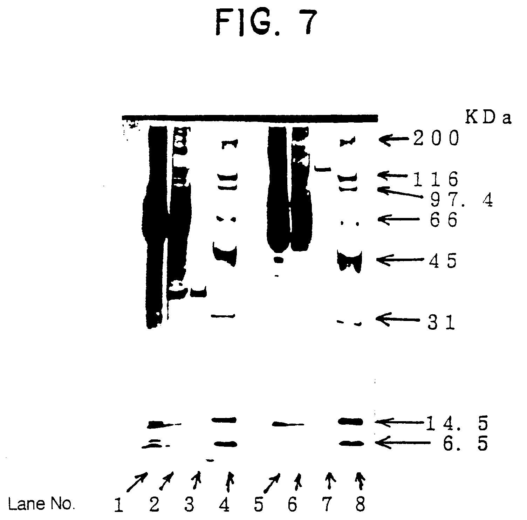

That the purified human chondrocalcin of this invention is a trimer can be ascertained by that the band of the reduction product thereof appears at the position corresponding to the molecular weight of about 1/3 of that of the non-reduced human chondrocalcin (see later-described examples and Fig.7). This reduction can be carried out by adding 0.5 % 2-mercaptoethanol to the above sample preparation solution and subjecting the mixture to incubation at 95° C for 5 minutes.

Thus the purified human chondrocalcin of this invention is one obtained by a genetic engineering method, and a highly pure one substantially not containing other proteins derived from human beings. Further, it is a highly pure one containing neither the monomer nor dimer of the peptide of the above amino acid sequence [I].

However, in the purified human chondrocalcin of this invention, an amino acid residue represented by X in the above amino acid sequence sometimes binds to the N-terminus side. This X is the amino acid residue of the sequence at the cleavage site in the case where the polypeptide of human chondrocalcin is translated and cleaved, and this amino acid residue sometimes remains at the N-terminus of the polypeptide (namely, the cases of m=1). The amino acid residue, therefore, should be an amino acid corresponding to the kind of signal peptide. Typical examples of the amino acid are Gln, Ala, Ser, Glu, Arg, Lys, Asp, Gly, etc.

The purified human chondrocalcin of this invention has immunological reactivity with an anti-bovine chondrocalcin antibody obtained by immunization using bovine chondrocalcin.

According to this invention, as described above, purified human chondrocalcin can be prepared by growing an eucaryote cell transformed with a vector containing a sequence which contains
(a) an eucaryotic promoter;
(b) optionally, an enhancer stimulating the above eucaryotic promoter; and
(c) the following DNA sequence [II] transcribed by the above eucaryotic promoter

   B-(C)ₘ-D [II]

   wherein, B, C, m and D have the same meanings as defined above,
   these sequences being arranged such that they can express human chondrocalcin, or an eucaryote cell infected with a virus containing the above sequence, and isolating secreted or accumulated human chondrocalcin.

In the sequence containing the above (a), (b) and (c), the DNA sequence of (c) is represented by the following formula [II].

B-(C)ₘ-D [II]

Here, B is a DNA sequence encoding an eucaryotic signal peptide, and this signal peptide can be any peptide acting as a signal peptide in an eucaryote cell in which the desired protein is expressed. An example of this signal peptide may be the signal peptide of human type II procollagen, and specifically, the following amino acid sequence [III] can be mentioned: 
Met-Ile-Arg-Leu-Gly-Ala-Pro-Gln-Ser-Leu-Val-Leu-Leu-Thr-
Leu-Leu-Val-Ala-Ala-Val-Leu-Arg-Cys-Gln-Gly ... [III]
As a DNA sequence encoding the above amino acid sequence [III], the following DNA sequence [IV] can be mentioned. 
ATGATTCGCC TCGGGGCTC CCCAGTCGC TGGTGCTGC TGACGCTGC
TCGTCGCCGC TGTCCTTCG GTGTCAGGG C ... [IV]
Further, in the DNA sequence of the above formula [II], C denotes a DNA sequence encoding the cleavage sequence, m denotes 0 or 1, preferably 1, and D denotes a DNA sequence encoding the above amino acid sequence P.

The C sequence is a DNA sequence encoding the cleavage sequence, and as a sequence usable for such purpose is desirable a sequence cleavable with the signal peptidase of the host cell, or an enzymatically cleavable sequence. As stated above, the amino acid of this cleavage sequence is an amino acid corresponding to the kind of signal peptide. For example, as the amino acid of the cleavage sequence, Gln can be mentioned, and as a DNA sequence encoding the above sequence, CAG can be mentioned.

The D sequence denotes the DNA sequence of the subunit of human chondrocalcin or a protein equivalent thereto. For example, the sequence D may be such a DNA sequence that part of the DNA was substituted, inserted or deleted, in such a range that the DNA sequence has substantially the same function as the DNA sequence has. The protein equivalent to the subunit of human chondrocalcin means one finally detectable by a method for immunological assay of human chondrocalcin.

As the sequence D, a DNA sequence encoding the above amino acid sequence P, which is a subunit of human chondrocalcin, can be mentioned, but conveniently, human cDNA can be used. The human cDNA is represented by the following formula. 
GACCAGGCAG CCGGTGGCCT GAGACAGCAT GACGCCGAGG
1 40
TGGATGCCAC ACTCAAGTCC CTCAACAACC AGATTGAGAG
80
CATCCGCAGC CCCGAGGGCT CCCGCAAGAA CCCTGCTCGC
120
ACCTGCAGAG ACCTGAAACT CTGCCACCCT GAGTGGAAGA
160
GTGGAGACTA CTGGATTGAC CCCAACCAAG GCTGCACCTT
200
GGACGCCATG AAGGTTTTCT GCAACATGGA GACTGGCGAG
240
ACTTGCGTCT ACCCCAATCC AGCAAACGTT CCCAAGAAGA
280
ACTGGTGGAG CAGCAAGAGC AAGGAGAAGA AACACATCTG
320
 
GTTTGGAGAA ACCATCAATG GTGGCTTCCA TTTCAGCTAT
360
GGAGATGACA ATCTGGCTCC CAACACTGCC AACGTCCAGA
400
TGACCTTCCT ACGCCTGCTG TCCACGGAAG GCTCCCAGAA
440
CATCACCTAC CACTGCAAGA ACAGCATTGC CTATCTGGAC
480
GAAGCAGCTG GCAACCTCAA GAAGGCCCTG CTCATCCAGG
520
GCTCCAATGA CGTGGAGATC CGGGCAGAGG GCAATAGCAG
560
GTTCACGTAC ACTGCCCTGA AGGATGGCTG CACGAAACAT
600
ACCGGTAAGT GGGGCAAGAC TGTTATCGAG TACCGGTCAC
640
AGAAGACCTC ACGCCTCCCC ATCATTGACA TTGCACCCAT
680
GGACATAGGA GGGCCCGAGC AGGAATTCGG TGTGGACATA
720
GGGCCGGTCT GCTTCTTG
738
As means for expressing the desired protein in eucaryote cells, many systems are well known per se.

For example, there can be mentioned, as a system expressing the protein in a yeast, "Expression of a polypeptide in a yeast" described in Japanese Published Unexamined Patent Application No. 57-159489; as a system expressing the protein in an insect cell, "A process for preparation of a recombinant baculovirus-expressing vector" described in Japanese Published Unexamined Patent Application No. 60-37988; and as a system expressing the protein in a mammalian cell, "Improvement of eucaryotic expression" described in Japanese Published Unexamined Patent Application No. 2-171198. But of course, there are many other systems.

A process for preparation of purified human chondrocalcin is described below, taking, as a typical example, a case where the baculovirus expression system exemplified above is used. In this case, the promoter which the baculovirus has is used as an eucaryotic promoter. In this connection, the promoter which a virus infecting to eucaryote cells has is an "eucaryotic promoter" because the promoter expresses the promoter function in eucaryote cells.

Further, the enhancer disposed so as to stimulate the promoter can also be disposed freely and readily by utilizing the enhancer of baculovirus, As to the most simple means for construction of them, such construction can be accomplished by utilizing a protein expression system, e.g. the polyhedrin gene, which the baculovirus has, and substitution-inserting an DNA sequence encoding the above amino acid sequence P, more preferably the DNA sequence represented by the above formula [II], into the polyhedrin gene region.

More specifically, the desired protein can be produced by using the intact EcoRI-I fragment [R. D. Posse et al., Virology, 185, 229-241 (1991)] part of Autographa california nuclear polyhedrosis virus (AcMNPV), preparing a variant virus in which the above DNA sequence has been substitution-inserted into its polyhedrin gene part, infecting an insect cell, for example an established strain SF-9 (ATCC CRL1711) of the Spodoptera frugiperda system with the variant virus, and culturing the infected cell.

The above variant virus can be conveniently prepared by homologous recombination, and a specific method therefor is also described detailedly in Japanese Published Unexamined Patent Application No. 60-37988. A baculovirus AcMNPV, preferably a baculovirus AclacZ, which has a marker inserted, a vector for homologous recombination and SF-9 strain are necessary as the starting materials for preparation of the above expression system. Such an expression system is sold as a kit such as MaxBac^{R} Baculovirus Expression system; INV IV-0822-04, supplied by Funakoshi Co., Ltd., and anyone can obtain it. Further, as to a baculovirus itself, it can also be obtained from nature according to a method described in G. E. Smith & M. D. Summers Virology, 89, 517-527 (1978), and insertion of a marker into it can be carried out according to a method by P. A. Kitts et al. [P.A. Kitts et al., Nucleic Acid Research 18 (1990) 5667-5672].

A vector for homologous recombination can also be obtained by, for example, inserting the EcoRI-I fragment of the above AcMNPV into the EcoRI site of pBR322, and substituting the DNA sequence represented by the above formula [II] for its polyhedrin gene portion.
The thus obtained vector for homologous recombination can be admixed with the above baculovirus with a marker AclacZ, and SF-9 culture cell can be cotransfected with the mixture. A virus group comprising a recombinant baculovirus and a nonrecombinant baculovirus are obtained by this operation. Usually, 10⁵ - 10⁶ pfu/ml of viruses exist in the supernatant of the third day after the transfection. When the supernatant is diluted so that 100 plaques may be formed per 35-ml dish, and assay is carried out, colorless and transparent plaques are formed in 1/2 to 1/3 of the dishes. These are selected as candidate strains for a recombinant baculovirus, and viruses are recovered. A DNA encoding human chondrocalcin is detected among these candidate strains according to the PCR method or the hybridization method to obtain a recombinant baculovirus. A large amount of the recombinant baculovirus can be prepared by taking a method comprising infecting again fresh SF-9 cells with the recombinant baculovirus.

The non-infected SF-9 cells in the above method can be cultured at 28° C in a medium containing 10 % bovine serum.

The SF-9 culture cells cultured and maintained in such medium is infected with the above recombinant baculovirus, and the protein is expressed. This can be accomplished by culturing the cells in the above medium or a serum-free medium at 28°C for about 96 hours.

The thus obtained culture broth contains the desired protein, AcMNPV, SF-9 cells, dead SF-9 cells, and DNA and proteins derived from SF-9 or AcMNPV. Therefore, in order to obtain the purified human chondrocalcin of this invention, human chondrocalcin is purified and separated from the above culture broth according to the following operations.

### Purification Process

(1) The cells are centrifuged.
(2) The virus is removed by ultrafiltration.
(3) Dialysis is carried out against 20mM potassium phosphate buffer (pH 6.5).
(4) A sample is loaded on a hydroxyapatite column equilibrated with 20mM potassium phosphate buffer (pH 6.5), washed with 200mM potassium phosphate buffer and eluted at a 200mM -> 1M concentration gradient of potassium phosphate buffers, and the main peak is collected.
(5) The main peak is dialyzed against H₂O, the solvent is removed by lyophilization, and the resultant solid is dissolved in 4M guanidine hydrochloride-150mM acetate buffer (pH 6.3).
(6) The solution obtained in (5) is fed on a Sephacryl S 200 gel filtration column, and effused with 4M guanidine hydrochloride-150mM acetate buffer (pH 6.3). Detection is carried out with OD280 and the main peak is collected.

The thus obtained purified human chondrocalcin exhibits one band in SDS-PAGE. Affinity column chromatography using an anti-bovine chondrocalcin monoclonal antibody can be added to the above purification steps.

Purified human chondrocalcin obtained by this invention can be utilized as a research reagent for research of pathology of diseases related to cartilages.

A polyclonal antibody and monoclonal antibodies against human chondrocalcin can be prepared using as an antigen the purified human chondrocalcin obtained by this invention, and therefore, the purified human chondrocalcin is useful as an immunogen. The thus obtained antibody is fit for use in immunological assay of human chondrocalcin in human specimens.

Further, purified human chondrocalcin obtained by this invention is indispensable also as a standard substance in an ELISA kit for immunoassay of human chondrocalcin.

Thus, according to this invention, in immunoassay reagents comprising an antibody recognizing human chondrocalcin, a standard substance and as required, a labeled antibody recognizing human chondrocalcin, a reagent for immunologically assaying human chondrocalcin in specimens, using purified human chondrocalcin as the standard substance is provided.

Further, according to this invention is provided a kit for immunoassay of human chondrocalcin in human specimens comprising the combination of
(a) a solid phase antibody wherein an antibody recognizing human chondrocalcin is bound,
(b) a labeled antibody recognizing human chondrocalcin,
(c) a solubilizer,
(d) a cleaning agent,
(e) a standard substance and
(f) a substrate for assaying the enzyme activity and a reaction-stopping agent, in the case where an antibody labeled with an enzyme is used, purified human chondrocalcin being used as said standard substance.

The antibody recognizing human chondrocalcin in the above reagent and kit can be a polyclonal or monoclonal antibody obtained by using as an immunogen purified human chondrocalcin provided by this invention.

### Example

This invention is detailedly described below by referring to specific examples, but not limited thereto.

### Example 1

### Preparation of a recombinant vector into which a cDNA encoding human chondrocalcin was inserted

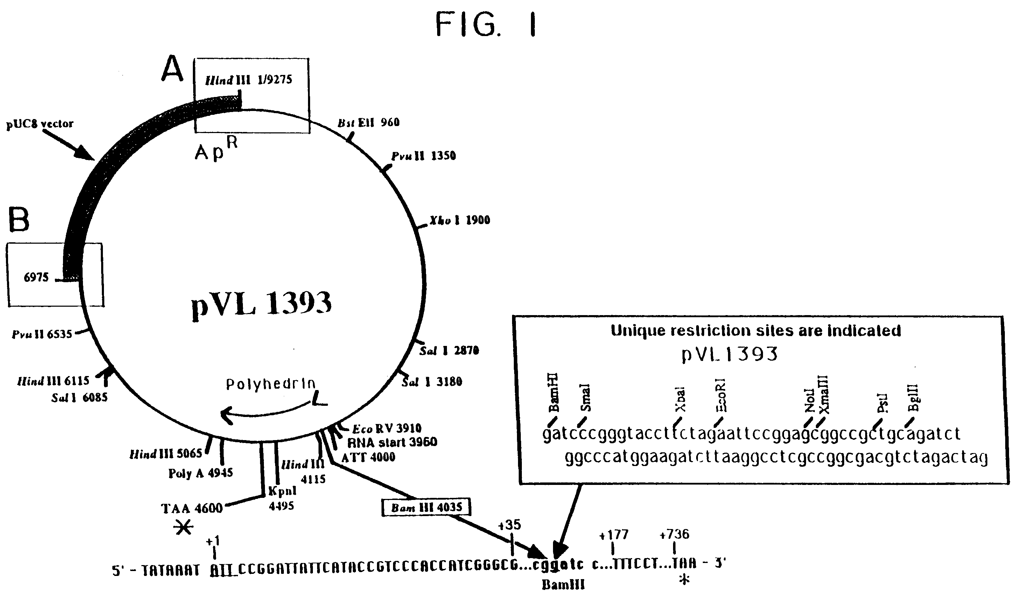

As an expression vector, pVL1393 (Invitrogen) was used [Technique, Vol. 2, No. 4, pp. 173-188 (1990); Virology, 185, pp. 229-241 (1991)]. As shown in Fig.1, this vector comprises pUC8 vector having inserted thereinto AcMNPV gene. These pUC8 vector and AcMNPV gene (Autographa california multiple nucleocapsid nuclear polyhedrosis virus gene) are introduced in the following literatures, respectively.

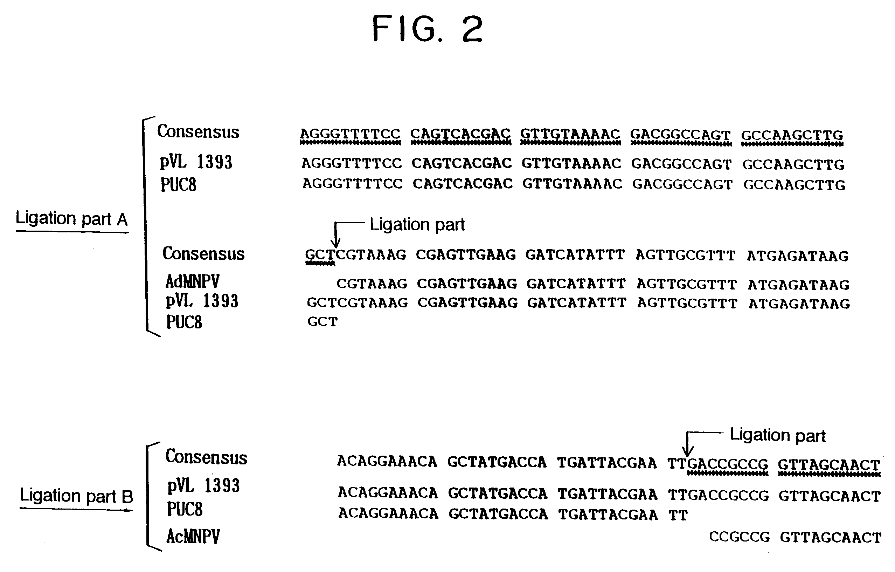

I. Vieira et al.; Gene 19, pp. 259-268 (1982) and
R. D. Posse et al., Virology, 185, pp. 229-241 (1991)]
The DNA sequences of the ligation parts of A and B in Fig. 1 in the above vector are shown in Fig. 2.

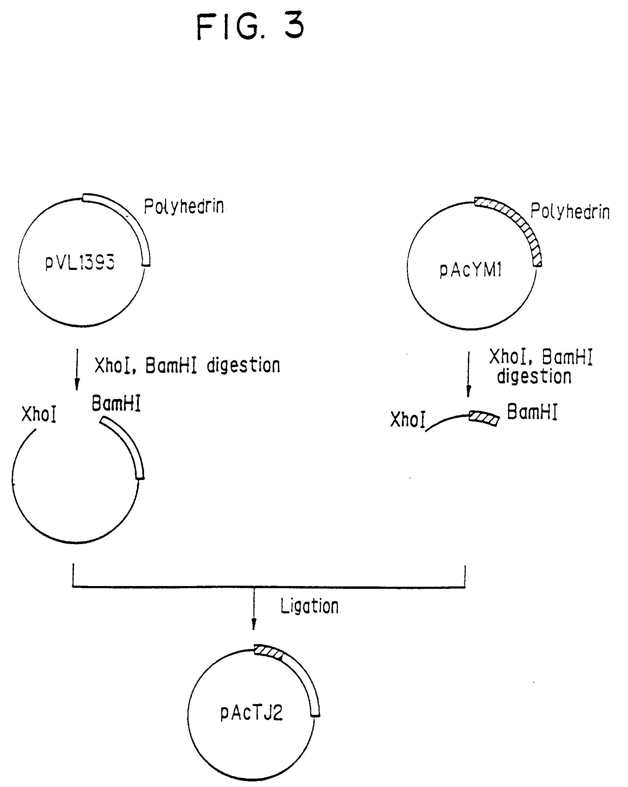

The vector pVL1393 shown in the above Fig. 1 was cleaved with XhoI and BamHI, and the 2.1kb XhoI-BamHI fragment of pAcYM1 [J. Genetical. Virology, 68, 1233-1250 (1987)] prepared by Matsuura et al. was inserted thereinto to give a vector which was named pAcTJ2 (see, Fig. 3). Further, human placental mRNA (Clonetech) was converted to cDNA, and human chondrocalcin expression DNA containing human chondrocalcin gene was amplified by PCR method as follows using the cDNA as a template.

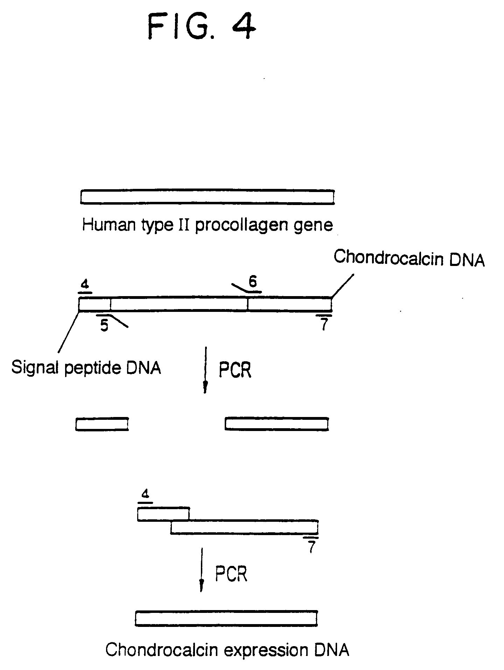

First, the signal peptide of human type II procollagen, represented by sequence number 3, was amplified by the PCR method using primers represented by sequence numbers 4 and 5, and a fragment of about 120bp was separated by agarose gel electrophoresis and recovered. Likewise, human chondrocalcin gene was amplified according to the PCR method using primers represented by sequence numbers 6 and 7, and a fragment of about 750bp was separated by agarose gel electrophoresis and recovered. Further, the human chondrocalcin expression DNA was amplified by the PCR method using these fragments as templates and using primers represented by Sequence numbers 4 and 7, and a fragment of about 800bp was separated by agarose gel electrophoresis, recovered and cleaved with BamHI and XbaI (Fig. 4).

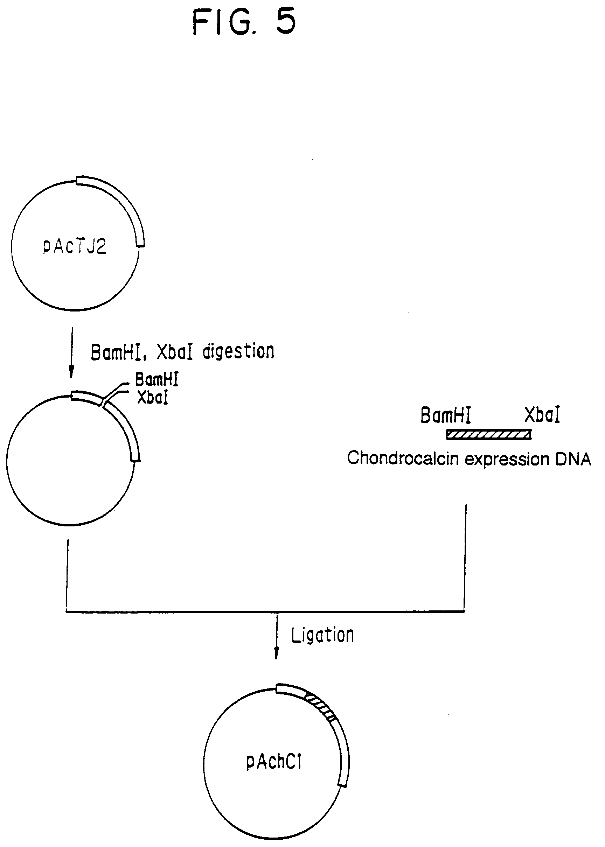

The thus obtained 825bp BamHI-XbaI fragment, and the recombinant vector pAcTJ2 cleaved with BamHI and XbaI were subjected to ligation reaction, and an Escherichia coli strain JM109 was transformed using the resultant vector. The plasmid was purified from several transformants (QIAGEN Plasmid midi kit was used) to give a homologous recombinant vector. This was named pAchC1 (Fig. 5).

### Example 2

### Preparation of a recombinant baculovirus

Preparation of a recombinant baculovirus was carried out according to the method of P. A. Kitts et al. [Nucleic Acid Research 18, 5667-5672 (1990)]. The DNA region encoding the polyhedrin of AcMNPV (virus genome DNA) was replaced by a gene encoding β-galactosidase to give a modified virus DNA:AclacZ.

14 ng of the product comprising this AclacZ whose one site had been cleaved with Eco81I to make it straight chain and 1 µg of pAchC1 were mixed, and the whole volume was made to be 8 µl with sterilized water. To this was added an equal amount of lipofectin (GIBCO Co.) having been diluted twice its volume with sterilized water, and the mixture was allowed to stand at room temperature for 15 minutes, and added to 1.5 ml of a serum-free medium EX-CELL400 (JRH Bio Science) containing 1 x 10⁶ cells of an insect cell SF-9 in a 35-ml dish. After 3 days culture, the medium was recovered and diluted properly, e.g. 10-fold or 100-fold, and SF-9 monolayer-cultured was infected with the dilution to form plaques. After 3 days culture, X-gal was added to the medium, and on the next day, plaques of the non-recombinant baculovirus stained in blue and plaques of the colorless and transparent recombinant baculovirus were separated. The colorless and transparent plaques were sucked up with a pasteur and suspended in a medium, the resultant virus solution was again properly diluted, and an insect cell was infected therewith and cultured. Purification was repeated until all the plaques thus-appearing became colorless and transparent. The thus obtained recombinant baculovirus was named AchC1.

### Example 3

### Preparation of human chondrocalcin using a recombinant baculovirus

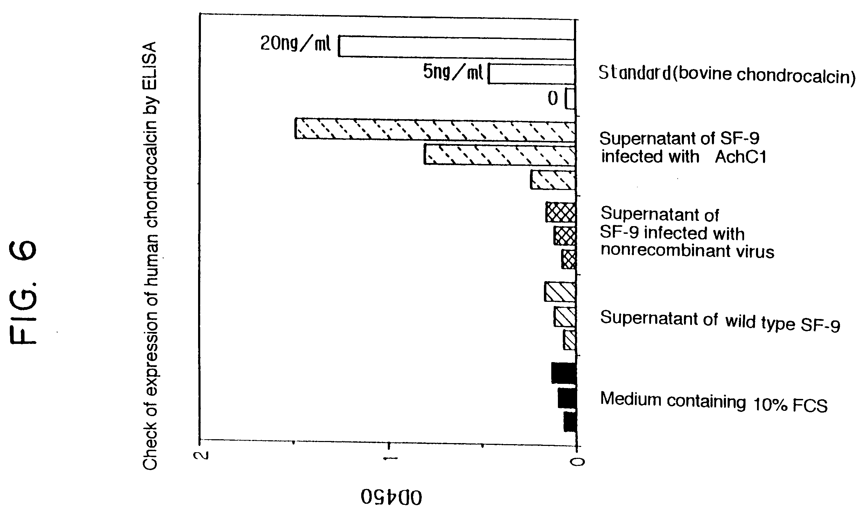

SF-9 cell was proliferated up to a density of 5 x 10⁶ cells/ml in a monolayer, the medium was removed, a serum-free medium containing 2 to 5 pfu of AchC1 per cell was added to infect the cell, and the cell was cultured for 4 days to secrete and express human chondrocalcin in the medium. The expressed protein was confirmed by ELISA using SDS-PAGE and an anti-bovine chondrocalcin antibody (Fig. 6).

The cells were removed by centrifugation (about 500 x g), the virus was removed by ultrafiltration (Fujifilter Filtron Miniset, fractional molecular weight 300kDa), and the resultant solution was dialyzed overnight against 20mM potassium phosphate buffer (pH 6.5). This solution was loaded on a hydroxyapatite column (Seikagaku Kogyo), the column was washed with 200mM potassium phosphate buffer, elution was carried out with a concentration gradient of 200mM -> 1M of potassium phosphate buffers. SDS-PAGE was carried out on each fraction to detect fractions which contain an eluted protein having a molecular weight of about 120kDa, and it was further confirmed, according to the method previously proposed by the present inventors (PCT Patent WO89-11097; "Reagent for Immunological Assay of Chondrocalcin"), that this 120kDa protein was human chondrocalcin.

The 120kDa protein-containing fractions were dialyzed against H₂O, freeze-dried to remove the solvent, dissolved in 4M guanidine hydrochloric acid-150mM acetate buffer (pH 6.3), and subjected for separation to Sephacryl S 200 gel filtration column (Pharmacia). The thus obtained purified human chondrocalcin exhibits one band in SDS-PAGE (Fig. 7).

### Reference Example 1

### Preparation of an anti-human chondrocalcin polyclonal antibody

An emulsion consisting of 1 mg of human chondrocalcin purified in Example 3 and complete Freund's adjuvant was subcutaneously administered to a rabbit. 19 Days later, 450 µg of human chondrocalcin was subcutaneously administered likewise. Further, 16 days later, an emulsion consisting of 450 µg of human chondrocalcin and complete Freund's adjuvant was subcutaneously administered. 10 Days later, blood was taken, the serum was separated therefrom, and its antibody titer against human chondrocalcin was assayed by enzyme immunoassay, namely by using human chondrocalcin as the antigen and horseradish peroxidase-labeled goat anti-rabbit IgG antibody as the second antibody, whereby it was confirmed that the antibody titer was 400,000-fold. Thereafter, the rabbit was exsanguinated, and the serum was separated from the blood taken, and purified by precipitation with 40 % saturated ammonium sulfate and purification with a protein A-Sepharose-4B column to give an anti-human chondrocalcin polyclonal antibody.

### Example 4

### Construction of a human chondrocalcin assay system

The F(ab')₂ of the anti-human chondrocalcin polyclonal antibody (anti-human chondrocalcin PCA) described in Reference Example 1 was bound to peroxidase in a conventional method to give a peroxidase-labeled F(ab')₂. Namely, 200 µl of 1M acetate buffer (pH 4.2) and 80 µg of pepsin having been dissolved in 40 µl of the same buffer were added to 2 ml of PBS solution of 2.0 mg/ml of the anti-human chondrocalcin PCA, and the mixture was subjected to reaction at 37°C for 4 hours. After completion of the reaction, the reaction solution was subjected to a Sephadex G-25 column (⌀ 2cm x 45cm) equilibrated with PBS, and the F(ab')₂ separated was recovered.

50 µl of a solution of 10 mg/ml MBS in dimethylformamide was added to 2 ml of a solution of 1 mg/ml the F(ab')₂ of anti-human chondrocalcin PCA, 0.01M phosphoric acid and 0.15M NaCl (pH 7.4), and the mixture was subjected to reaction at a temperature of 25°C for 30 minutes. The reaction solution was then gel-filtered using a column packed with Sephadex G-25 and 0.1M phosphate buffer (0.1M PB)(pH 6.0) to separate the maleimidized antibody and unreacted MBS.

On the other hand, 120 µl of 60 mg/ml dimethylformamide solution of S-acetylmercaptosuccinic anhydride was added to 2 ml of 0.1M PB solution (pH 6.5) of 10 mg/ml HRP, the mixture was subjected to reaction at 25°C for 2 hours. Then, 800 µl of 0.1M Tris-hydrochloric acid buffer (pH 7.0). 160 µl of 0.1M EDTA and 1.6 ml of 1M hydroxylamine were added, and the mixture was subjected to reaction at 0°C for 4 minutes. The reaction solution was then put in a collodion bag, and dialyzed at 4°C for 3 days using a 0.1M PB (pH 6.0) and 5mM EDTA-containing solution to give thiolized HRP.

Then, 2 mg of the maleimidized antibody and 4 mg of the thiolized HRP were mixed, and the mixture was concentrated under ice cooling using a collodion bag until a protein concentration of 4 to 10 mg/ml was attained, and allowed to stand overnight at 15 to 20°C. The solution was gel-filtered using a column packed with Ultrogel AcA44 (produced by Pharmacia LKB Co.) to give HRP-labeled anti-human chondrocalcin PCA F(ab')₂. This labeled F(ab')₂ was used for preparation of the following calibration curve.

Anti-human chondrocalcin PCA described in Example 4 was made into a solution in 0.1M phosphate/citrate buffer (pH 3.5) having its concentration of 20 µg/ml, polystyrene beads (diameter 6 mm) sufficiently washed were put in the solution, and the mixture was allowed to stand all day and night at 4°C to carry out immobilization. The beads were washed with PBS, allowed to stand in 1.0% BSA-PBS solution all day and night at 4°C to carry out the after-coat, and washed with PBS to give beads having immobilized thereon anti-human chondrocalcin PCA.

A series of dilutions containing 0, 0.625, 1.25, 2.5 and 5 ng/ml purified human chondrocalcin were prepared with 1.0% BSA-final concentration 0.1% skim milk-PBS (pH 7.2), 50 µl each of the dilutions were put in test tubes, respectively, and 150 µl portions of 1.0% BSA-0.1% skim milk-PBS (pH 7.2) were added. The HRP-labeled anti-human chondrocalcin PCA F(ab')₂ was diluted with 1.0% BSA-final concentration 0.1% skim milk-PBS (pH 7.2) so that the concentration of the F(ab')₂ component could be 0.7 µg/ml, 200 µl portions of the resultant labeled F(ab')₂ solution were put in test tubes, respectively, portions of the beads having immobilized thereon anti-human chondrocalcin PCA were added, respectively, and the mixtures were subjected to reaction at 37°C for 90 minutes. After washing the beads with PBS-0.05% Tween-20, 400 µl portions of a substrate solution for HRP (2.5mM H₂O₂, 0.0225% 3,3',5,5'-tetramethylbenzyne) were added, the mixtures were subjected to coloring reaction at 37°C for 30 minutes, 1,000 µl portions of 1N sulfuric acid solution were added, respectively to discontinue the reaction, and absorbance was measured at 450 nm using a spectrophotometer.

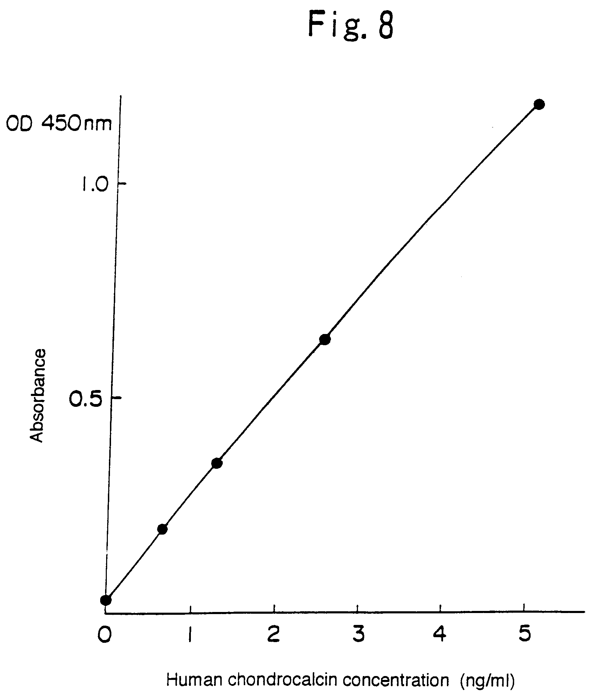

The resultant calibration curve was shown in Fig. 8.

As apparent from Fig. 8, the measurement lower limit was 10 pg/tube, and this assay system exhibited remarkably higher sensitivity than the radioimmunoassay by A. Hinek et al. where the measurement lower limit was 100 ng/ml [A. Hinek et al., J. Cell Biol., 104, 1435-1441 (1987)].

### Example 5

### Assay of specimens (joint humor, serum) by the human chondrocalcin assay system

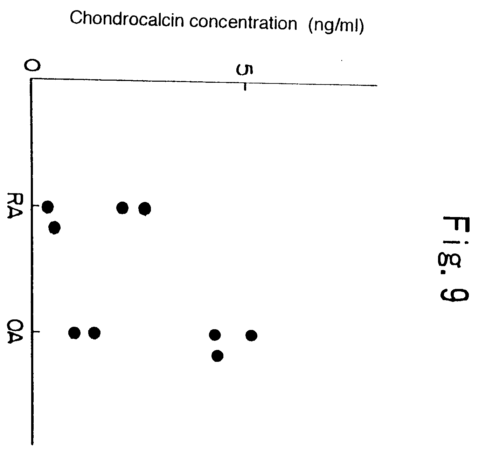

(a) 50 µl each of the joint humors of patients of chronic articular rheumatism (RA) (4 specimens) and patients of osteoarthritis (OA) (5 specimens) were put in test tubes, respectively, 150 µl portions of an assay buffer [1.0% BSA-0.1% skim milk-PBS (pH 7.2)] were added, 200 µl portions of 0.7 µg/ml the anti-human chondrocalcin F(ab')₂ HRP-labeled antibody were added, portions of the beads having immobilized thereon anti-human chondrocalcin antibody were added, respectively, and the mixtures were subjected to incubation at 37°C for 90 minutes.
After washing the beads with PBS-0.05% Tween-20, 400 µl portions of the substrate solution for HRP were added, respectively, the mixtures were subjected to coloring reaction at 37°C for 30 minutes, 1,000 µl portions of 1N sulfuric acid solution were added, respectively to discontinue the reaction, and absorbance was measured at 450 nm using a spectrophotometer.
The calibration curve therefor was prepared by diluting human chondrocalcin with the assay buffer to 0, 0.625, 1.25, 2.5 and 5 ng/ml, putting 50 µl each of the dilutions into test tubes, respectively, and thereafter making the same operations as in Example 4.
The results were shown in the following table and also in Fig. 9.

| Specimen | OD₄₅₀ | Concentration (ng/ml) |
|---|---|---|
| RA1 | 0.551 | 2.13 |
| " 2 | 0.672 | 2.65 |
| " 3 | 0.119 | 0.37 |
| " 4 | 0.158 | 0.52 |
| OA1 | 0.403 | 1.50 |
| " 2 | 1.033 | 4.30 |
| " 3 | 0.290 | 1.03 |
| " 4 | 0.210 | 5.12 |
| " 5 | 1.043 | 4.35 |

(b) 25 µl each of sera taken from infants were put in test tubes, respectively, 175 µl portions of the assay buffer were added, and thereafter the same operations as in (a) were carried out for assay.

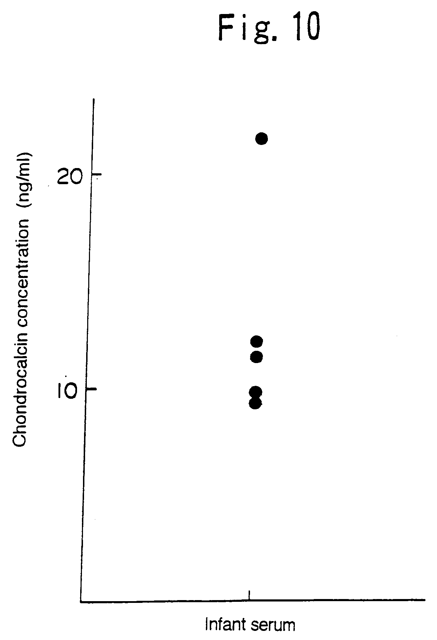

The results were shown in the following table and also in Fig. 10.

| Specimen | OD₄₅₀ | Concentration (ng/ml) |
|---|---|---|
| 1 | 1.356 | 11.98 |
| 2 | 1.098 | 9.22 |
| 3 | 1.348 | 11.34 |
| 4 | 1.155 | 9.76 |
| 5 | 2.486 | 21.47 |

### Brief Description of Drawings

Fig. 1 is a drawing showing the restriction enzyme map of the vector pVL1393 and the insertion sites of the pUC8 vector and the AcMNPV gene.

Fig. 2 shows the DNA sequences at the ligation part of the insertion sites of the pUC8 vector and the AcMNPV gene.

Fig. 3 is a schematic drawing on preparation of a homologous recombinant vector pAcTJ2.

Fig. 4 is the method of preparing a human chondrocalcin expression DNA.

Fig. 5 is a schematic drawing on preparation of a homologous recombinant vector pAchC1 for expression of human chondrocalcin.

Fig. 6 is a graph showing ascertainment of the expression of human chondrocalcin with ELISA.

Fig. 7 is a chart showing the purity assay of human chondrocalcin with SDS-PAGE (electrophoresis).

Fig. 8 shows a calibration curve for an immunoassay system prepared using an anti-human chondrocalcin antibody.

Fig. 9 shows results obtained when the joint humors of patients of chronic articular rheumatism (RA) and patients of osteoarthritis (OA) were used as specimens and human chondrocalcin in the specimens was assayed according to immunoassay using purified human chondrocalcin as a standard substance.

Fig. 10 shows results obtained when human chondrocalcin in the sera of infants was assayed according to immunoassay.

### Description of Symbols

In Fig. 7, the respective Lane Nos. show the following meanings.
1: Culture supernatant (reduction) of wild type SF9
2: Culture supernatant (reduction) of SF9 infected with Achc1
3: Purified human chondrocalcin (reduction)
5: Culture supernatant (non-reduction) of wild type SF9
6: Culture supernatant (non-reduction) of SF9 infected with Achc1
7: Purified human chondrocalcin (non-reduction)
4 and 8: Molecular-weight markers

[Sequence table]
Sequence No. 1
Length of sequence: 246
Type of sequence: amino acid
Topology: straight-chain
Kind of sequence: protein
Sequence:

Asp Gln Ala Ala Gly Gly Leu Arg Gln His Asp Ala Glu Val Asp
1               5                   10                  15
Ala Thr Leu Lys Ser Leu Asn Asn Gln Ile Glu Ser Ile Arg Ser
                20                  25                  30
Pro Glu Gly Ser Arg Lys Asn Pro Ala Arg Thr Cys Arg Asp Leu
                35                  40                  45
Lys Leu Cys His Pro Glu Trp Lys Ser Gly Asp Tyr Trp Ile Asp
                50                  55                  60
Pro Asn Gln Gly Cys Thr Leu Asp Ala Met Lys Val Phe Cys Asn
                65                  70                  75
Met Glu Thr Gly Glu Thr Cys Val Tyr Pro Asn Pro Ala Asn Val
                80                  85                  90
Pro Lys Lys Asn Trp Trp Ser Ser Lys Ser Lys Glu Lys Lys His
                95                  100                 105
Ile Trp Phe Gly Glu Thr Ile Asn Gly Gly Phe His Phe Ser Tyr
                110                 115                 120
Gly Asp Asp Asn Leu Ala Pro Asn Thr Ala Asn Val Gln Met Thr
                125                 130                 135
Phe Leu Arg Leu Leu Ser Thr Glu Gly Ser Gln Asn Ile Thr Tyr
                140                 145                 150
His Cys Lys Asn Ser Ile Ala Tyr Leu Asp Glu Ala Ala Gly Asn
                155                 160                 165

Leu Lys Lys Ala Leu Leu Ile Gln Gly Ser Asn Asp Val Glu Ile
                170                 175                 180
Arg Ala Glu Gly Asn Ser Arg Phe Thr Tyr Thr Ala Leu Lys Asp
                185                 190                 195
Gly Cys Thr Lys His Thr Gly Lys Trp Gly Lys Thr Val Ile Glu
                200                 205                 210
Tyr Arg Ser Gln Lys Thr Ser Arg Leu Pro Ile Ile Asp Ile Ala
                215                 220                 225
Pro Met Asp Ile Gly Gly Pro Glu Gln Glu Phe Gly Val Asp Ile
                230                 235                 240
Gly Pro Val Cys Phe Leu
                245
Sequence No. 2
Length of sequence: 738
Type of sequence: nucleic acid
Topology: straight-chain
Kind of sequence
Fragment type
Sequence:
GACCAGGCAG CCGGTGGCCT GAGACAGCAT GACGCCGAGG
1 40
TGGATGCCAC ACTCAAGTCC CTCAACAACC AGATTGAGAG
80
CATCCGCAGC CCCGAGGGCT CCCGCAAGAA CCCTGCTCGC
120
ACCTGCAGAG ACCTGAAACT CTGCCACCCT GAGTGGAAGA
160
GTGGAGACTA CTGGATTGAC CCCAACCAAG GCTGCACCTT

200
GGACGCCATG AAGGTTTTCT GCAACATGGA GACTGGCGAG
240
ACTTGCGTCT ACCCCAATCC AGCAAACGTT CCCAAGAAGA
280
ACTGGTGGAG CAGCAAGAGC AAGGAGAAGA AACACATCTG
320
GTTTGGAGAA ACCATCAATG GTGGCTTCCA TTTCAGCTAT
360
GGAGATGACA ATCTGGCTCC CAACACTGCC AACGTCCAGA
400
TGACCTTCCT ACGCCTGCTG TCCACGGAAG GCTCCCAGAA
440
CATCACCTAC CACTGCAAGA ACAGCATTGC CTATCTGGAC
480
GAAGCAGCTG GCAACCTCAA GAAGGCCCTG CTCATCCAGG
520
GCTCCAATGA CGTGGAGATC CGGGCAGAGG GCAATAGCAG
560
GTTCACGTAC ACTGCCCTGA AGGATGGCTG CACGAAACAT
600
ACCGGTAAGT GGGGCAAGAC TGTTATCGAG TACCGGTCAC
640
AGAAGACCTC ACGCCTCCCC ATCATTGACA TTGCACCCAT
680
GGACATAGGA GGGCCCGAGC AGGAATTCGG TGTGGACATA
720
GGGCCGGTCT GCTTCTTG
738

Sequence No. 3
Length of sequence: 25
Type of sequence: amino acid
Topology: straight-chain
Kind of sequence
Fragment type
Sequence:
Met Ile Arg Leu Gly Ala Pro Gln Ser Leu Val Leu Leu Thr Leu
1               5                   10                  15
Leu Val Ala Ala Val Leu Arg Cys Gln Gly
                20                  25
Sequence No. 4
Length of sequence: 28
Type of sequence: nucleic acid
Topology: straight-chain
Kind of sequence
Fragment type
Sequence:
AAGGATCCAT GATTCGCCTC GGGGCTCC

Sequence No. 5
Length of sequence: 57
Type of sequence: nucleic acid
Topology: straight-chain
Kind of sequence
Fragment type
Sequence:
CTCGGCGTCA TGCTGTCTCA GGCCACCGGC TGCCTGGTCC
TGGCCCTGAC ACCGAAG

Sequence No. 6
Length of sequence: 57
Type of sequence: nucleic acid
Topology: straight-chain

Kind of sequence
Fragment type
Sequence:
ACGCTGCTCG TCGCCGCTGT CCTTCGGTGT CAGGGCCAGG
ACCAGGCAGC CGGTGGC

Sequence No. 7
Length of sequence: 38
Type of sequence: nucleic acid
Topology: straight-chain
Kind of sequence
Fragment type
Sequence:
TTTTTTTTTC TAGATTACAA GAAGCAGACC GGCCCTAT

## Claims

1. Purified human chondrocalcin comprising a trimer of a polypeptide having the following amino acid sequence [I]
Xₘ - P [I]
wherein
X denotes an amino acid residue derived from a cleavage sequence,
m denotes 0 or 1, and
P denotes the following amino acid sequence:

Asp Gln Ala Ala Gly Gly Leu Arg Gln His Asp Ala Glu Val Asp
1               5                   10                  15
Ala Thr Leu Lys Ser Leu Asn Asn Gln Ile Glu Ser Ile Arg Ser
                20                  25                  30
Pro Glu Gly Ser Arg Lys Asn Pro Ala Arg Thr Cys Arg Asp Leu
                35                  40                  45
Lys Leu Cys His Pro Glu Trp Lys Ser Gly Asp Tyr Trp Ile Asp
                50                  55                  60
Pro Asn Gln Gly Cys Thr Leu Asp Ala Met Lys Val Phe Cys Asn
                65                  70                  75
Met Glu Thr Gly Glu Thr Cys Val Tyr Pro Asn Pro Ala Asn Val
                80                  85                  90
Pro Lys Lys Asn Trp Trp Ser Ser Lys Ser Lys Glu Lys Lys His
                95                  100                 105
Ile Trp Phe Gly Glu Thr Ile Asn Gly Gly Phe His Phe Ser Tyr
                110                 115                 120
Gly Asp Asp Asn Leu Ala Pro Asn Thr Ala Asn Val Gln Met Thr
                125                 130                 135
Phe Leu Arg Leu Leu Ser Thr Glu Gly Ser Gln Asn Ile Thr Tyr
                140                 145                 150

His Cys Lys Asn Ser Ile Ala Tyr Leu Asp Glu Ala Ala Gly Asn
                155                 160                 165
Leu Lys Lys Ala Leu Leu Ile Gln Gly Ser Asn Asp Val Glu Ile
                170                 175                 180
Arg Ala Glu Gly Asn Ser Arg Phe Thr Tyr Thr Ala Leu Lys Asp
                185                 190                 195
Gly Cys Thr Lys His Thr Gly Lys Trp Gly Lys Thr Val Ile Glu
                200                 205                 210
Tyr Arg Ser Gln Lys Thr Ser Arg Leu Pro Ile Ile Asp Ile Ala
                215                 220                 225
Pro Met Asp Ile Gly Gly Pro Glu Gln Glu Phe Gly Val Asp Ile
                230                 235                 240
Gly Pro Val Cys Phe Leu
                245

2. The purified human chondrocalcin according to claim 1, which immunologically reacts with an anti-bovine chondrocalcin antibody.

3. The purified human chondrocalcin according to claim 1, which is obtained by recombinant gene manipulation.

4. A process for preparing purified human chondrocalcin which comprises growing an eucaryote cell transformed or infected with a vector or virus containing a sequence which contains
(a) an eucaryotic promoter;
(b) optionally, an enhancer stimulating the above eucaryotic promoter; and
(c) the following DNA sequence [II] transcribed by the above eucaryotic promoter
B - (C)ₘ - D [II]
wherein
B denotes a DNA sequence encoding an eucaryotic signal peptide,
C denotes a DNA sequence encoding the amino acid residue X in the above formula [I]
m denotes 0 or 1, and
D denotes a DNA sequence encoding the amino acid sequence P in the above formula [I], these sequences being arranged such that they can express human chondrocalcin,
and isolating secreted or accumulated human chondrocalcin.

5. The process for preparing purified human chondrocalcin according to claim 4 wherein said eucaryotic signal peptide is represented by the following amino acid sequence [III]

Met Ile Arg Leu Gly Ala Pro Gln Ser Leu Val Leu Leu Thr Leu
1               5                   10                  15
Leu Val Ala Ala Val Leu Arg Cys Gln Gly
                20                  25

6. The process for preparing purified human chondrocalcin according to claim 4 wherein said eucaryotic cell is an insect cell which can be infected with a baculovirus.

7. The process for preparing purified human chondrocalcin according to claim 4 wherein said DNA sequence encoding the amino acid sequence in the above formula [I] is the following DNA sequence.

GACCAGGCAG CCGGTGGCCT GAGACAGCAT GACGCCGAGG
1 40
TGGATGCCAC ACTCAAGTCC CTCAACAACC AGATTGAGAG
80
CATCCGCAGC CCCGAGGGCT CCCGCAAGAA CCCTGCTCGC
120
ACCTGCAGAG ACCTGAAACT CTGCCACCCT GAGTGGAAGA
160

GTGGAGACTA CTGGATTGAC CCCAACCAAG GCTGCACCTT
200
GGACGCCATG AAGGTTTTCT GCAACATGGA GACTGGCGAG
240
ACTTGCGTCT ACCCCAATCC AGCAAACGTT CCCAAGAAGA
280
ACTGGTGGAG CAGCAAGAGC AAGGAGAAGA AACACATCTG
320
GTTTGGAGAA ACCATCAATG GTGGCTTCCA TTTCAGCTAT
360
GGAGATGACA ATCTGGCTCC CAACACTGCC AACGTCCAGA
400
TGACCTTCCT ACGCCTGCTG TCCACGGAAG GCTCCCAGAA
440
CATCACCTAC CACTGCAAGA ACAGCATTGC CTATCTGGAC
480
GAAGCAGCTG GCAACCTCAA GAAGGCCCTG CTCATCCAGG
520
GCTCCAATGA CGTGGAGATC CGGGCAGAGG GCAATAGCAG
560
GTTCACGTAC ACTGCCCTGA AGGATGGCTG CACGAAACAT
600
ACCGGTAAGT GGGGCAAGAC TGTTATCGAG TACCGGTCAC
640
AGAAGACCTC ACGCCTCCCC ATCATTGACA TTGCACCCAT
680
GGACATAGGA GGGCCCGAGC AGGAATTCGG TGTGGACATA
720
GGGCCGGTCT GCTTCTTG
738

8. The process for preparing purified human chondrocalcin according to claim 4 wherein said DNA sequence encoding the eucaryotic signal peptide is the following DNA sequence.

ATGATTCGCC TCGGGGCTC CCCAGTCGC TGGTGCTGC TGACGCTGC
TCGTCGCCGC TGTCCTTCG GTGTCAGGG C

9. In immunoassay reagents each comprising an antibody recognizing human chondrocalcin, a standard substance and, as required, a labeled antibody recognizing human chondrocalcin, a reagent for immunologically assaying human chondrocalcin in specimens, using purified human chondrocalcin as the standard substance.

10. A kit for immunoassay of human chondrocalcin in human specimens comprising the combination of
(a) a solid phase antibody wherein an antibody recognizing human chondrocalcin is bound,
(b) a labeled antibody recognizing human chondrocalcin,
(c) a solubilizer,
(d) a cleaning agent,
(e) a standard substance and
(f) a substrate for assaying the enzyme activity and a reaction-stopping agent, in the case where an antibody labeled with an enzyme is used, purified human chondrocalcin being used as said standard substance.

11. Use of purified human chondrocalcin for immunoassay of human chondrocalcin in specimens.
